# EUROPEAN PATENT APPLICATION

(11) **EP 4 732 892 A1**
(43) Date of publication of application: **29.04.2026**
(21) Application number: 25203118.2
(22) Date of filing: 18.09.2025
(51) Int. Cl.: A61N 1/368, A61N 1/37, A61N 1/08

(54) **IMPLANTABLE MEDICAL DEVICE EMPLOYING ELECTRODE INTEGRITY MONITORING**

(30) Priority: 22.10.2024 EP 24208029
(71) Applicant: BIOTRONIK SE & Co. KG, 12359 Berlin (DE)
(72) Inventor: Fischer, René, 10245 Berlin (DE)
(74) Representative: Biotronik Corporate Services SE

(57) **Abstract**

The present invention relates to an implantable medical device (1) for stimulating a human or animal heart. During operation, the implantable medical device (1) executes a method comprising the following steps: a) detecting a right ventricular electric signal, the detected right ventricular electric signal comprising a plurality of right ventricular events (13); b) detecting a left ventricular electric signal, the detected left ventricular electric signal comprising a plurality of left ventricular events (16); c) defining one of the right ventricular electric signal and the left ventricular electric signal as reference signal (15) and the other as supervised signal (18); d) counting a number of ventricular events (13, 16) in the supervised signal (18) occurring during a predeterminable number of ventricular events (16, 13) in the reference signal (15); e) determining whether there is a deviation between the number of ventricular events (13, 16) in the supervised signal (18) and the predeterminable number of ventricular events (16, 13) in the reference signal (15); f) if the deviation between the number of ventricular events (13, 16) in the supervised signal (18) and the predeterminable number of ventricular events (16, 13) in the reference signal (15) is bigger than a threshold, i) perform a storing of at least a part of the reference signal (15) and/or of at least a part of the supervised signal (18) in the memory unit (33) to allow a later evaluation of the stored part (21, 22) of the reference signal (15) and/or of the supervised signal (18) and/or ii) to increment a counter for each deviation that is bigger than the threshold and to store the incremented counter and/or iii) sending a notification via the data communication unit (36) to an external device or remote system.

## Description

The present invention relates to an implantable medical device according to the preamble of claim 1 and to a method for operating an implantable medical device according to the preamble of claim 13.

Prior art describes a plurality of possibilities to detect electrode integrity problems (electrode malfunctions) and/or electrode dislodgement of an implantable medical device for cardiac stimulation.

The present disclosure uses the term "electrode" for describing means for electrical stimulation, wherein an electrode comprises at least one electrode pole, and whereby electrical stimulation pulses are delivered via the electrode poles. For instance, the electrode can be an electrode lead. The term "electrode integrity" is used as describing the proper mechanical and electrical functionality of the electrode and shall also include the proper attachment of the electrode at the designated site. Thus, an electrode having an integrity issue shall describe an electrode having a mechanical or electrical malfunction, and/or a dislodged electrode.

According to an impedance-based approach, exceeding an absolute threshold or falling below an absolute threshold during painless monitoring of the shock and stimulation activity of an implantable medical device is used to detect an electrode integrity and/or electrode dislodgement problem. Furthermore, a quasi-continuous impedance monitoring can be performed. Then, conspicuous, strong impedance changes on a sample-to-sample basis at a comparatively high sampling rate can provide information on an electrode integrity and/or electrode dislodgement problem.

According to a timing-based approach, a short interval counter can be applied. An increased number of unphysiological short intervals (having a duration less than 140 ms) triggers further evaluation of the detected signal or directly indicates a suspected electrode integrity problem. Other timing patterns in the detected electric signal can also be used for triggering further evaluation of the detected signal.

While impedance-based approaches provide a strong indicator that an electrode integrity problem exists, they allow a continuous monitoring of the electrode integrity only under significantly increased power consumption. Exclusively time-based approaches, on the other hand, require that the electrode fault can be detected as oversensing in a bipolar derivation, typically between the right ventricular tip electrode pole and the right ventricular ring electrode pole of the used electrode.

It is an object of the present invention to provide a continuous energy-saving monitoring of cardiac electric signals to identify indicators for electrode integrity problems and to allow a later thorough analysis of the respective signals.

This object is achieved with an implantable medical device for stimulating a human or animal heart according to claim 1. Such an implantable medical device comprises a processor, a memory unit, a stimulation unit, and a detection unit. The stimulation unit serves for stimulating a human or animal heart. The detection unit serves for detecting an electric signal of the same heart, i.e., a cardiac electric signal. The detection unit comprises a first electrode for detecting right ventricular electric signals and a second electrode for detecting left ventricular electric signals. In this context, the first electrode comprises a first electrode pole and a second electrode pole. Likewise, the second electrode comprises a third electrode pole and a fourth electrode pole.

According to an aspect of the present invention, the memory unit comprises a computer-readable program that causes the processor to perform the steps explained in the following when being executed on the processor.

In a method step, a right ventricular electric signal is detected with the first electrode. In this context, the detected right ventricular electric signal comprises a plurality of right ventricular events. An appropriate example of such a right ventricular electric signal is an intracardiac electrogram (IEGM).

In another method step, a left ventricular electric signal is detected with the second electrode. In this context, the detected left ventricular electric signal comprises a plurality of left ventricular events.

In another method step, the right ventricular electric signal or the left ventricular electric signal is defined as reference signal. The respective other signal is defined as supervised signal. Thus, it is possible to define the right ventricular electric signal as reference signal and the left ventricular electric signal as supervised signal. Likewise, it is possible to define the left ventricular electric signal as reference signal and the right ventricular electric signal as supervised signal.

In another method step, the supervised signal and the reference signal are monitored during a time period covering a predeterminable number of ventricular events in the reference signal. The number of ventricular events in the supervised signal occurring in this time period is counted.

In another method step, a deviation between the number of ventricular events in the supervised signal and the predeterminable number of ventricular events in the reference signal is determined. If the heart is beating regularly and if the first electrode and the second electrode do not show any electrode integrity problems, there is no deviation between the counted number of ventricular events in the supervised signal and the predeterminable number of ventricular events in the reference signal. However, if a deviation could be detected, this indicates that there might be an electrode integrity problem.

In another method step, it is checked whether the deviation between the number of ventricular events in the supervised signal and the predeterminable number of ventricular events in the reference signal is bigger than a threshold. If this is the case, action is taken to be able to evaluate the origin of the deviation between the number of ventricular events in the supervised signal and in the reference signal. For this purpose, a storing of at least a part of the reference signal and/or of at least a part of the supervised signal in the memory unit is performed. This storing allows a later evaluation of the stored part of the reference signal and/or of the supervised signal, e.g., in a separate evaluation unit. For instance, the stored part of the reference signal and/or of the supervised signal is sent to a remote server for further evaluation. Additionally or alternatively, a counter for each deviation that is bigger than the threshold is incremented and the incremented counter is stored. Such a comparably simple counter is highly appropriate for evaluating behavior trends of the implantable medical device and its electrodes over a predeterminable period of time and thus provides additional analytic functions with respect to electrode integrity problems. Additionally or alternatively, a notification is sent via the data communication unit to an external device or remote system. The notification is reviewed by a user on an interface, for instance on the external device or any other device where the notification is forwarded to by the remote system. According to an embodiment, the notification is sent via the data communication unit to an external device or remote system when the counter exceeds a counter threshold. That counter threshold can be predefined in a way that a crossing of the counter threshold indicates an electrode integrity issue.

This implantable medical device represents a highly performant system for early detection of electrode integrity problems (electrode failures) based on an evaluation of the detected right ventricular signal and the detected left ventricular electric signal, e.g., a detected intracardiac electrogram (IEGM). The implantable medical device does not require a specific design of its system components. They can be chosen and operated such that the overall sensitivity and the overall specificity can be adjusted to the respective needs of the intended kind of application of the implantable medical device. Thus, irrespective of enabling a detailed analysis of suspicious areas of the detected cardiac electric signal to detect electrode integrity problems, the implantable medical device enables a 24/7 monitoring of a patient's heart (i.e., it can be operated with a high overall sensitivity). In addition, it does not require a complex sorting-out of false-positive trigger events for storing the detected cardiac electric signal that takes place when applying more demanding approaches or a higher calculation complexity based on a predeterminable dataset (i.e., the implantable medical device can be operated with a high specificity).

It is not important for the presently claimed and described subject matter how the stored cardiac electric signal is finally evaluated or analyzed. Rather, the presently described and claimed solution merely aims in providing sensible and reliable causes of triggering a further event (follow-up action). One possible follow-up action is the storing of at least a part of the detected cardiac electric signal in case of a suspected electrode integrity problem. The sored signal can afterwards be analyzed to find out whether there is indeed an electrode integrity problem. Another possible follow-up action is the increment of the counter for each deviation that is bigger than the threshold and the storing of the incremented counter.

In an embodiment, the implantable medical device is an implantable pulse generator (IPG), an implantable cardioverter-defibrillator (ICD), or a device for cardiac resynchronization therapy (CRT).

In an embodiment, the predeterminable number of ventricular events in the reference signal lies in a range from 2 to 100, in particular from 3 to 95, in particular from 4 to 90, in particular from 5 to 85, in particular from 6 to 80, in particular from 7 to 75, in particular from 8 to 70, in particular from 9 to 65, in particular from 10 to 60, in particular from 11 to 55, in particular from 12 to 50, in particular from 13 to 45, in particular from 14 to 40, in particular from 15 to 35, in particular from 16 to 30, in particular from 17 to 25, in particular from 18 to 20. A number lying in a range from 3 to 7 is particularly appropriate.

In an embodiment, a pacing event being visible in the supervised signal is not counted as ventricular event. In doing so, an undesired deviation between the number of ventricular events in the supervised signal and the number of ventricular events in the reference signal is avoided that would not be due to an electrode integrity problem, but rather due to a cardiac stimulation.

In an embodiment, the computer-readable program causes the processor to generate a timeout event under certain circumstances. Such a timeout event is deemed to be a ventricular event in the reference signal. Such a timeout event is generated in case that no ventricular event has been detected in the reference signal during a predeterminable first time period. Such a non-detection of ventricular events in the reference signal during the predeterminable first time period indicates that the electrode that serves for providing the electric signals for the reference signal is dislocated or has another electrode integrity problem so that it is not able to properly detect any more ventricular events. Since such non-enablement can be of temporarily limited duration, the generation of the timeout event bridges such periods of non-enablement. The predeterminable first time period is typically chosen such that a non-occurrence of any ventricular events in that first time period would correspond to an unphysiological low cardiac rate. To give an example, a cardiac rate of approximately 45 beats per minutes results in 6 ventricular events within 8 seconds. If no ventricular event at all is detected within 8 seconds, this clearly indicates that the number of detected cardiac events is far too low. Thus, the generation of the timeout event serves to deal with undersensing by the electrode that is responsible for delivering the reference signal.

In an embodiment, the computer-readable program causes the processor to repeatedly generate the timeout event with a predeterminable repetition rate in case that no ventricular event has been detected in the reference signal during the predeterminable first time period. Such a repeated generation of the timeout events serves for bridging the duration of an undersensing scenario of the electrode that is responsible for delivering the reference signal. To give an example, the timeout event can be generated every 1 to 2.5 seconds, in particular every 1.5 to 2 seconds. Then, a cardiac rate between 24 bpm and 60 bpm would be simulated in the reference signal, and a minimum rate of 24 to 60 updates per minute of the proposed method is enabled.

In an embodiment, the computer-readable program causes the processor to store the number of ventricular events detected in the supervised signal during a single interval of the reference signal as a value in a ring buffer, in particular in a rolling ring buffer. Such a ring buffer can store a predeterminable number of values, wherein the oldest value is discarded once the latest value is stored. In doing so, the ring buffer always contains the same number of values (n), wherein the n^{th} latest values are stored in the ring buffer.

In an embodiment, the ring buffer serves for storing a number of values that corresponds to the predeterminable number of ventricular events in the reference signal. In particular, the number of values to be stored in the ring buffer is by one lower than the predeterminable number of ventricular events in the reference signal. To give an example, if the supervised signal shall be monitored over a time period that is needed for six ventricular events to occur in the reference signal, the ring buffer would be able, in this embodiment, to store five values (since six ventricular events result in five ventricular intervals). Each stored value indicates the number of ventricular events detected in the supervised signal during the respective ventricular interval of the reference signal.

In an embodiment, the computer-readable program causes the processor to store the latest 5 to 180 seconds, in particular the latest 10 to 150 seconds, in particular the latest 15 to 120 seconds, in particular the latest 20 to 100 seconds, in particular the latest 25 to 80 seconds, in particular the latest 25 to 60 seconds, in particular the latest 30 to 40 seconds of the reference signal and/or of the supervised signal prior to determining whether the deviation exceeds the threshold. Typically, such range of the detected cardiac electric signal is fully sufficient to evaluate by a subsequent analysis whether there is indeed an electrode integrity problem as assumed by the internal evaluation of the events of the cardiac electric signal.

In an embodiment, the computer-readable program causes the processor to store the next 1 to 60 seconds, in particular the next 2 to 50 seconds, in particular the next 3 to 40 seconds, in particular the next 4 to 30 seconds, in particular the next 5 to 20 seconds, in particular the next 6 to 10 seconds, preferably the next 30 to 60 seconds of the reference signal and/or of the supervised signal upon determining that the deviation exceeds the threshold. Thus, in this embodiment, the implantable medical device does not only store the history of the triggering ventricular event but also the post-history. This allows an even better subsequent evaluation of the cardiac electric signal and a distinction between an unusual course of the signal due to a physiologic reason and an unusual course of the signal due to the suspected electrode integrity problem.

In an embodiment, the implantable medical device does not only store the detected cardiac electric signal in the memory unit upon receiving the trigger signal upon recognizing a sufficiently high deviation between the number of ventricular events in the reference signal and in the supervised signal, but also additional information such as marker time points and marker types. This additional information is stored, in an embodiment, in a data-reduced or compressed manner to save storing space and to reduce the power being necessary for storing, reading and/or transferring such additional data.

In an embodiment, the memory unit comprises a ring buffer for short-term caching the reference signal and/or the supervised signal, as well as a long-term memory, wherein storing of at least a part of the reference signal and/or the supervised signal comprises writing the part of the reference signal and/or of the supervised signal to be stored into the long-term memory. This guarantees that the stored part of the detected cardiac electric signal is available at a later time for evaluation remote from the implantable medical device. The long-term memory can be generally a volatile or a non-volatile memory. To reduce the risk of data loss, it is typically a non-volatile memory.

In an embodiment, the implantable medical device comprises a data communication unit that enables a data transfer of data stored within the memory unit to an external device or remote system. Such external device is, in an embodiment, a telemedicine system such as a home monitoring system. The data transferred from the implantable medical device to this remote system can then be evaluated on the remote system. To allow a better visualization for the medical staff engaged with the evaluation of the data, the events in the cardiac electric signals having suspicious features can be displayed in a highlighted manner.

In an embodiment, a data transfer takes place immediately after having stored the part of the cardiac electric signal into the memory unit. In an embodiment, the stored cardiac electric signal is transferred to the remote system upon the next opportunity to do so, i.e., upon confirmed contact with a transfer unit forming part of a data transfer network and/or being operatively coupled with the remote monitoring system.

In an embodiment, the data communication unit serves for transferring data to the remote system in a wireless manner. All standard data transmission protocols or specifications are appropriate for such a wireless data communication. Examples of standard data transmission protocols or specifications are the Medical Device Radiocommunications Service (MICS), the Bluetooth Low Energy (BLE) protocol, the Zigbee specification, the long range wide area network (LoRaWAN) protocol, the wireless personal area network (WPAN) specification, the low-power wide-area network (LPWAN) specification, the wireless local area network (WLAN) specification, the Global System for Mobile Communications (GSM) specification, the Long-Term Evolution (LTE) standard, and the fifth-generation technology standard for broadband cellular networks (5G).

In an embodiment, the counter is a day counter configured to store a number of deviations per day that is bigger than the threshold. Such a day counter makes it particularly easy to evaluate a trend or development of the deviation between the reference signal and the supervised signal over an extended period of time in daily intervals. This helps to evaluate whether there is a general trend of an electrode integrity problem or whether an observed electrode integrity problem can be classified as singular event that does not indicate an electrode integrity problem in general.

In an embodiment, the computer-readable program causes the processor to use the right ventricular electric signal as reference signal and the left ventricular electric signal at supervised signal. In this embodiment, the computer-readable program causes the processor likewise to use the left ventricular electric signal as reference signal and the right ventricular electric signal as supervised signal in a parallel evaluation of an identical temporal section of the right ventricular electric signal and of the left ventricular electric signal. Thus, in this embodiment, a cross-evaluation takes place in which each signal serves as reference signal and as supervised signal at the same time. While the computational requirement of such cross-evaluation is somewhat higher than the evaluation of a single signal as supervised signal, the cross-evaluation gives additional insight in any potential electrode integrity problem and thus helps to detect such electrode integrity problem significantly earlier. Using the proposed method and implantable medical device, electrode integrity issues and electrode dislodgement / dislocation of an electrode configured to stimulate the right ventricle and/or an electrode configured to stimulate the left ventricular electrode can be detected by monitoring the described cross-evaluation and monitoring a counter which is incremented if the deviation between the number of ventricular events in a supervised signal and the predeterminable number of ventricular events in a reference signal is bigger than a threshold.

In an aspect, the present invention relates to a method for operating an implantable medical device for stimulating a human or animal heart, in particular an implantable medical device according to the preceding explanations. This method comprises the steps explained in the following.

In a method step, a right ventricular electric signal is detected with a first electrode. In this context, the detected right ventricular electric signal comprises a plurality of right ventricular events.

In another method step, a left ventricular electric signal is detected with a second electrode. In this context, the detected left ventricular electric signal comprises a plurality of left ventricular events.

In another method step, the right ventricular electric signal or the left ventricular electric signal is defined as reference signal. The respective other signal is defined as supervised signal. Thus, it is possible to define the right ventricular electric signal as reference signal and the left ventricular electric signal as supervised signal. Likewise, it is possible to define the left ventricular electric signal as reference signal and the right ventricular electric signal as supervised signal.

In another method step, the supervised signal and the reference signal are monitored during a time period covering a predeterminable number of ventricular events in the reference signal. The number of ventricular events in the supervised signal occurring in this time period is counted.

In another method step, a deviation between the number of ventricular events in the supervised signal and the predeterminable number of ventricular events in the reference signal is determined. If a deviation is detected, this indicates that there might be an electrode integrity problem.

In another method step, it is checked whether the deviation between the number of ventricular events in the supervised signal and the predeterminable number of ventricular events in the reference signal is bigger than a threshold. If this is the case, action is taken to be able to evaluate the origin of the deviation between the number of ventricular events in the supervised signal and in the reference signal. For this purpose, a storing of at least a part of the reference signal and/or of at least a part of the supervised signal in the memory unit is performed. This storing allows a later evaluation of the stored part of the reference signal and/or of the supervised signal, e.g., in a separate evaluation unit. For instance, the stored part of the reference signal and/or of the supervised signal is sent to a remote server for further evaluation. Additionally or alternatively, a counter for each deviation that is bigger than the threshold is incremented and the incremented counter is stored. Such a comparably simple counter is highly appropriate for evaluating behavior trends of the implantable medical device and its electrodes over a predeterminable period of time and thus provides additional analytic functions with respect to electrode integrity problems. Additionally or alternatively, a notification is sent via the data communication unit to an external device or remote system. The notification is reviewed by a user on an interface, for instance on the external device or any other device where the notification is forwarded to by the remote system. According to an embodiment, the notification is sent via the data communication unit to an external device or remote system when the counter exceeds a counter threshold. That counter threshold can be predefined in a way that a crossing of the counter threshold indicates an electrode integrity issue.

In an embodiment, the counter is a day counter configured to store a number of deviations per day that is bigger than the threshold. In addition, a progression of the day counter over a course of days is monitored and/or analyzed in this embodiment. This helps to better classify any observed electrode integrity problem as expressed by the deviation between the supervised signal and the reference signal.

All embodiments of the implantable medical device can be combined in any desired way and can be transferred either individually or in any arbitrary combination to the described method. Likewise, all embodiments of the described method can be combined in any desired way and can be transferred either individually or in any arbitrary combination to the implantable medical device.

Further details of aspects of the present invention will be explained in the following making reference to exemplary embodiments and accompanying Figures. In the Figures:
- Figure 1A: schematically shows a system comprising an implantable medical device;
- Figure 1B: schematically shows different components of the implantable medical device of Figure 1A;
- Figure 2: shows two electric deviations obtained along the first and second sensing vectors illustrated in Figure 1A;
- Figure 3A: shows a schematic flowchart of an embodiment of a method employed by an implantable medical device;
- Figure 3B: shows a schematic depiction of an operational mode employed by the same implantable medical device; and
- Figure 4: is a schematic illustration of the stored parts of the detected reference signal and supervised signal.

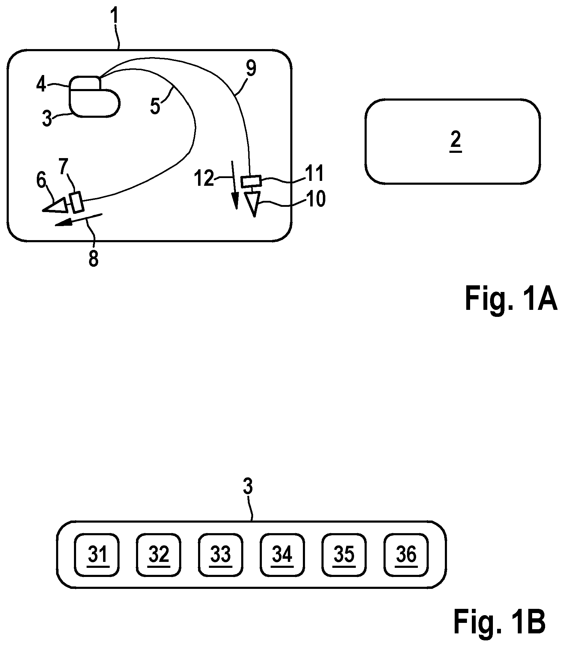

Figure 1A shows a system comprising a device for cardiac resynchronization therapy (CRT) 1 as example of an implantable medical device for stimulating the human or animal heart. The system further comprises a home monitoring system 2 serving as remote system. It is possible for the CRT device 1 to establish a wireless data communication with the home monitoring system 2.

The CRT device 1 comprises a housing 3 with a header 4 and a first electrode 5 connected to the header 4. The first electrode 5 comprises a first tip electrode pole 6 and a first ring electrode pole 7 that is proximally arranged from the first tip electrode pole 6. A first sensing vector 8 is defined from the first ring electrode pole 7 to the first tip electrode pole 6. Electric cardiac signals sensed between the first tip electrode pole 6 and first the ring electrode pole 7, i.e., along the first sensing vector 8, are directly recorded within a heart chamber, typically the right ventricle. Thus, the first electrode 5 is designed and arranged to sense right ventricular electric signals.

The CRT device 1 additionally comprises a second electrode 9 that is also connected to the header 4. The second electrode 9 comprises a second tip electrode pole 10 and a second ring electrode pole 11 that is proximally arranged from the second tip electrode pole 10. A second sensing vector 12 is defined from the second ring electrode pole 11 to the second tip electrode pole 10. Electric cardiac signals sensed between the second tip electrode pole 10 and the second ring electrode pole 11, i.e., along the second sensing vector 12, are also directly recorded within a heart chamber, typically the left ventricle. Thus, the second electrode 9 is designed and arranged to sense left ventricular electric signals.

Figure 1B schematically illustrates individual components of the CRT device 1 that are comprised within the housing 3. In this and in all following Figures, similar elements will be denoted with the same reference numeral. The housing 3 houses a detection unit 31 (also referred to as sensing unit) that comprises an analog-to-digital converter, a bandpass filter, and an offset compensation. The detection unit 31 is operatively connected with a processor 32 that has access to a memory unit 33. The memory unit 33 serves for storing instructions for the processor 32 as well as data detected by the detection unit 31. The housing 3 further comprises an evaluation unit 34 that can also be part of the processor 32 and that serves for extracting features from the detected cardiac electric signal. The housing 3 further comprises a stimulation unit 35 that serves for stimulating the heart from which the detection unit 31 detects electric signals. The first electrode 5 along with its first tip electrode pole 6 and first ring electrode pole 7 as well as the second electrode 9 along with its second tip electrode 10 and second ring electrode 11 (confer Figure 1A) form part of the detection unit 31 and of the stimulation unit 35. Additionally, the housing 3 comprises a data communication unit 36 that serves for data transfer to the home monitoring system 2 (confer Figure 1A).

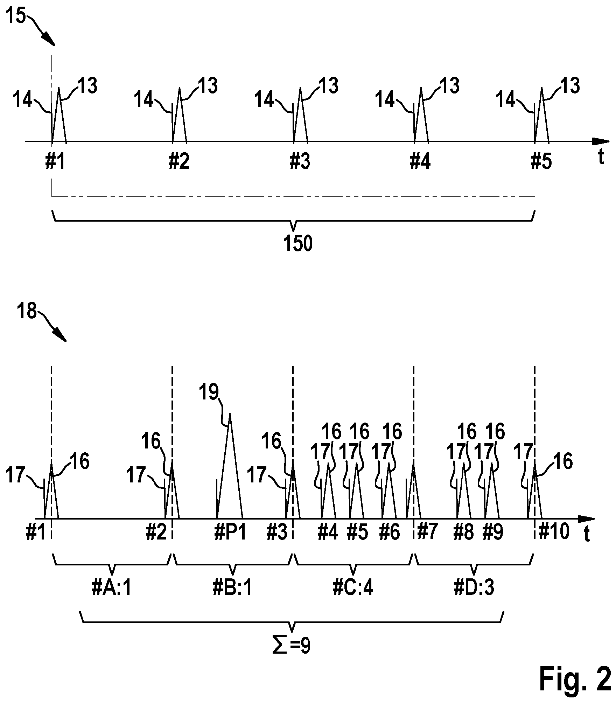

Figure 2 shows, in its upper panel, an electric derivation obtained along the first sensing vector 9 illustrated in Figure 1A. The sensing unit 31 (confer Figure 1B) has detected five different ventricular events 13 and has assigned an event sensing signal 14 (additionally marked with consecutive numbers #1 to #5) to each of these events 13. In this context, the individual event sensing signals 14 are placed at the beginning of each of the events 13. The electric derivation obtained along the first sensing vector 9 represents a right ventricular electric signal so that each of the events 13 is a right ventricular event. The electric derivation comprising the right ventricular events 13 serves as reference signal 15. It comprises a number 150 of right ventricular (reference) event sensing signals 14, wherein this number 150 amounts to five in the example of Figure 2.

Figure 2 shows, in its lower panel, an electric derivation obtained during the same time period as the derivation shown in the upper panel of Figure 2. However, the derivation of the lower panel is obtained along the second sensing vector 12 and constitutes a left ventricular electric signal comprising a plurality of different left ventricular events 16. A left ventricular event sensing signal 17 (additionally marked with consecutive numbers #1 to #10) has been assigned to each of the left ventricular events 16. The individual left ventricular event sensing signals 17 are placed at the beginning of each of the left ventricular events 16. Dashed vertical lines are placed in the lower panel of Figure 2 at those time points at which the reference event sensing signals 14 were set in the upper panel of Figure 2.

The electric derivation comprising the left ventricular events 16 serves as supervised signal 18. It comprises a sum Σ of left-ventricular (supervised) event sensing signals 17, wherein this sum Σ amounts to nine in the example of Figure 5. It should be noted that the sum Σ is calculated for exactly that time period that starts with the first reference event sensing signal 14 and that ends with the last reference event sensing signal 14 of the time period to be monitored.

In this time period, there are four intervals (marked with #A, #B, #C, and #D). The interval #A extends from the first reference event sensing signal 14 to the second reference event sensing signal 14 and comprises one supervised event sensing signal 17.

The interval #B extends from the second reference event sensing signal 14 to the third reference event sensing signal 14 and also comprises one supervised event sensing signal 17. In addition, it comprises one pacing event 19 that is, however, not calculated as supervised sensing signal 17.

The interval #C extends from the third reference event sensing signal 14 to the fourth reference event sensing signal 14 and comprises four supervised event sensing signals 14.

Finally, the interval #D extends from the fourth reference event sensing signal 14 to the fifth reference event sensing signal 14 and comprises three supervised event sensing signals 17. Consequently, the sum of the values of the supervised event sensing signals 17 detected in the individual intervals #A, #B, #C, and #D amounts to nine (1+1+4+3).

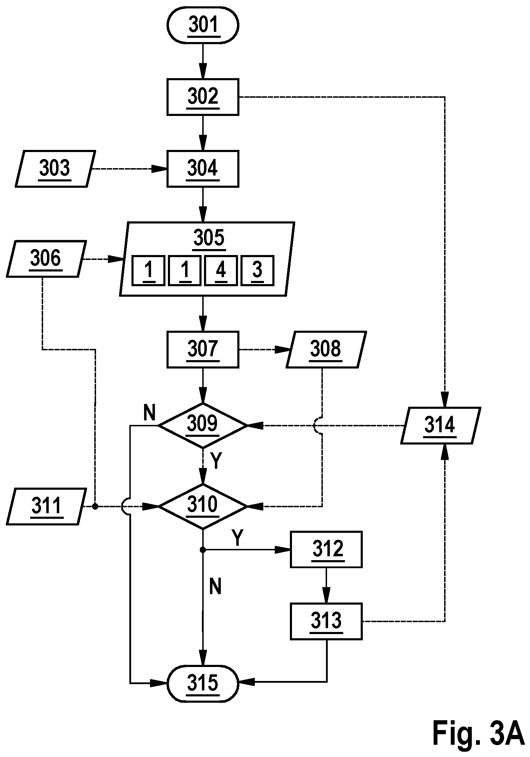

Figure 3A is a schematic flowchart of a method applied in an embodiment of the implantable medical device. Reference is also made to the previously explained Figures, in particular to Figure 2 the embodiment of which will be further elucidated by the flowchart of Figure 3A.

In an assignment step 301, a reference event sensing signal is assigned to a detected ventricular event. This results in an increment of a reference event counter by 1 in an increment step 302. The assigned reference event sensing signal terminates a cardiac interval. The number of events detected in the supervised channel in the terminated interval is determined in a determination step 303. This determined number is stored in a storage step 304 in a ring buffer 305. In the embodiment shown in Figure 3A, the ring buffer 305 comprises four elements (1, 1, 4, 3), i.e., a number of elements that corresponds to the number of supervised event sensing signals determined in the embodiment illustrated in Figure 2. The ring buffer 305 is able to store exactly four numbers. This configuration of the ring buffer 305 is achieved in a ring buffer configuration step 306. It is obvious that the number of reference events that determines the number of values that can be stored in the ring buffer 305 and that is provided to the ring buffer 305 in the ring buffer configuration step 306 can differ from the number four that is used in the present example.

Upon storing the latest value in the ring buffer 305 in the storage step 304, an update of the sum of supervised event sensing signals assigned to the determined supervised ventricular events is done in an update step 307. After this update step 307, the sum amounts to 9 in the present example. This value is assigned in a sum assignment step 308 to a respective parameter.

In a first decision step 309, it is determined whether the reference event counter is equal to or bigger than the number of reference events that was assigned to the ring buffer 305 in the ring buffer configuration step 306. If this is the case (Y, for yes), it is determined in a second determination step 310 whether the sum of supervised events exceeds the predeterminable threshold. This predeterminable threshold is provided in a threshold provision step 311 to the second decision step 310. In the example shown in Figure 3 A, the threshold amounts to 6. Since the sum of 9 is higher than 6 (Y), a trigger event is detected in the trigger event detection step 312. Due to this detection of the trigger event, a trigger is released.

This trigger can have various purposes. Here, the trigger serves for storing at least a part of the reference signal and/or at least a part of the supervised signal in the memory unit of the implantable medical device. Alternatively or additionally, the trigger serves for incrementing a counter for each deviation between the reference signal and the supervised signal that is bigger than the threshold to store the incremented counter. These steps triggered after reaching the trigger event detection step 312 are not illustrated in Figure 3A.

After the trigger event detection step 312, the reference event counter is reset to 0 in a resetting step 313. After this, the method directly proceeds to the termination step 315. The reset reference event counter is then reintroduced into the method via a reference event counter provision step 314. Likewise, the incremented reference event counter resulting from the increment step 302 is provided to other method steps via the reference counter provision step 314.

If it turns out in the first decision step 309 that the reference event counter is smaller than the number of reference events (N, for no), the method directly proceeds from the first decision step 309 to the termination step 315. If, in the second decision step 310, the sum of the supervised events is not bigger than the threshold (N), the check for the sum of the supervised events for this iteration ends in termination step 315.

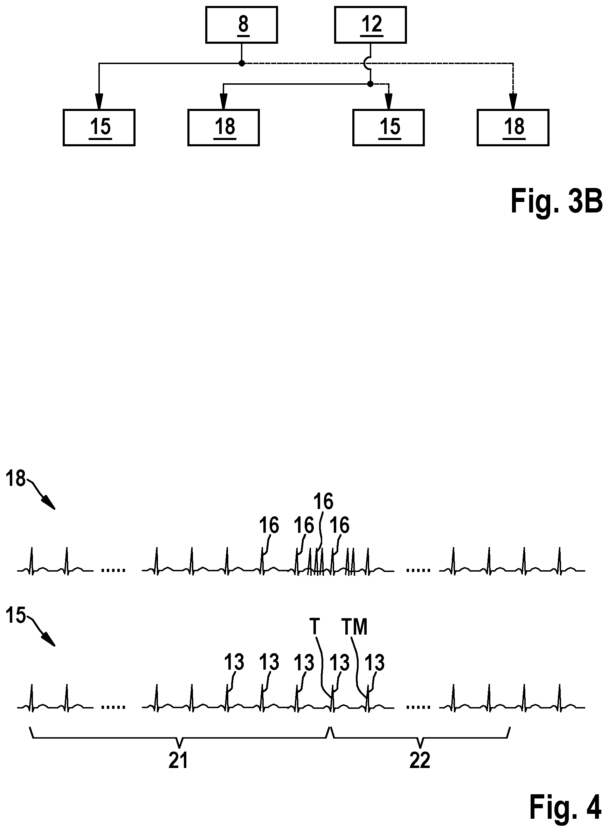

Figure 3B schematically illustrates an operational mode of the implantable medical device the method of which was explained with respect to Figure 3A. According to this operational mode, an electric derivation along the first sensing vector 8 serves as reference signal 15 for a first evaluation, wherein an electric derivation along the second sensing vector 12 serves as supervised signal 18 for this first evaluation. At the same time, the same electric derivation along the first electric sensing vector 8 serves as supervised signal 18 for a second evaluation, wherein the same electric derivation along the second sensing vector 12 serves as reference signal 15 for this second evaluation. The first evaluation and the second evaluation take place at the same time and comprise the same temporal section of the electric derivations along the first sensing vector 8 and the second sensing vector 12. Due to this cross-evaluation, a particularly sensitive detection of any oversensing event and any undersensing event being indicative for electrode integrity problems is made possible.

Thus, for the presently described implantable medical device and the methods implemented by this implantable medical device, it makes no difference if the right ventricular signal or the left ventricular signal is used as reference signal. Rather, both the right ventricular signal and the left ventricular signal are likewise appropriate to be used as reference signal for the respective other signal. At the same time, both the right ventricular signal and the left ventricular signal are likewise appropriate to be used as supervised signal for the respective other signal as reference signal.

Figure 4 shows, in its upper panel, a supervised signal 18 comprising a plurality of supervised ventricular events 16, only some of which are marked with the respective reference numeral (confer Figure 2 for more details). In its lower panel, Figure 4 shows a reference signal 15 comprising a plurality of reference ventricular events 13, only some of which are marked with the respective reference numeral (confer Figure 2 for more details).

One of the reference ventricular events 13 is additionally marked with the letter T since it constitutes a trigger signal at which the condition that the sum of the supervised signals exceeds the threshold of six for the last four reference events is fulfilled (confer in this respect also Figure 3A for more details). Thus, the reference ventricular event T serves for triggering the trigger events explained above, e.g., storing at least a part of the reference signal 15 and of the supervised signal 18.

For this purpose, both the history 21 and post-history 22 of the reference signal 15 with respect to the trigger signal T is stored. In this context, both the reference signal 15 and the supervised signal 18 are stored over the same time period, i.e., over the same part of the history 21 the post-history 22. Such a part of the detected cardiac electric signal is typically sufficient to subsequently analyze the signal more thoroughly for an electrode integrity problem. This is typically done in the remote monitoring system 2 (confer Figure 1A) since this remote monitoring system 2 allows computationally more complex feature extractions and classifications from the stored cardiac electric signal than this is possible within the CRT device 1.

The reference ventricular event 13 succeeding the trigger signal T (i.e., the reference ventricular even T+1) does not serve as trigger signal and does not trigger any subsequent action since the reference event counter has just been reset in the resetting step 313 (confer Figure 3A for more details). Thus, the reference event signal T+1 only serves for incrementing the reference event counter by 1 (and thus to 1).

### List of reference numerals

- 1: Device for cardiac resynchronization therapy (CRT)
- 2: Home monitoring system
- 3: Housing
- 4: Header
- 5: First electrode
- 6: First tip electrode pole
- 7: First ring electrode pole
- 8: First sensing vector
- 9: Second electrode
- 10: Second tip electrode pole
- 11: Second ring electrode pole
- 12: Second sensing vector
- 13: Right ventricular event
- 14: Right ventricular event sensing signal
- 15: Reference signal
- 16: Left ventricular event
- 17: Left ventricular event sensing signal
- 18: Supervised signal
- 21: History of the detected cardiac electric signal
- 22: Post-history of the detected cardiac electric signal
- 31: Detection unit
- 32: Processor
- 33: Memory unit
- 34: Evaluation unit
- 35: Stimulation unit
- 36: Data communication unit
- 301: Assignment step
- 302: Increment step
- 303: Determination step
- 304: Storage step

- 305: Ring buffer
- 306: Ring buffer configuration step
- 307: Update step
- 308: Sum assignment step
- 309: First decision step
- 310: Second decision step
- 311: Threshold provision step
- 312: Trigger event detection step
- 313: Resetting step
- 314: Reference event counter provision step

## Claims

1. Implantable medical device (1) for stimulating a human or animal heart, comprising a processor (32), a memory unit (33), a stimulation unit (34) configured to stimulate a human or animal heart, and a detection unit (31) configured to detect an electric signal of the same heart, wherein the detection unit (31) comprises a first electrode (5) for detecting right ventricular electric signals and a second electrode (9) for detecting left ventricular electric signals, wherein the first electrode (5) comprises a first electrode pole (6) and a second electrode pole (7) and wherein the second electrode (9) comprises a third electrode pole (10) and a fourth electrode pole (11),
**characterized**
**in that** the memory unit (33) comprises a computer-readable program that causes the processor (32) to perform the following steps when being executed on the processor (32):
a) detecting a right ventricular electric signal with the first electrode (5), the detected right ventricular electric signal comprising a plurality of right ventricular events (13);
b) detecting a left ventricular electric signal with the second electrode (9), the detected left ventricular electric signal comprising a plurality of left ventricular events (16);
c) defining one of the right ventricular electric signal and the left ventricular electric signal as reference signal (15) and the other as supervised signal (18);
d) counting a number of ventricular events (13, 16) in the supervised signal (18) occurring during a predeterminable number of ventricular events (16, 13) in the reference signal (15);
e) determining whether there is a deviation between the number of ventricular events (13, 16) in the supervised signal (18) and the predeterminable number of ventricular events (16, 13) in the reference signal (15);
f) if the deviation between the number of ventricular events (13, 16) in the supervised signal (18) and the predeterminable number of ventricular events (16, 13) in the reference signal (15) is bigger than a threshold, i) perform a storing of at least a part of the reference signal (15) and/or of at least a part of the supervised signal (18) in the memory unit (33) to allow a later evaluation of the stored part (21, 22) of the reference signal (15) and/or of the supervised signal (18) and/or ii) increment a counter for each deviation that is bigger than the threshold and to store the incremented counter and/or iii) sending a notification via the data communication unit (36) to an external device or remote system.

2. Implantable medical device according to claim 1, **characterized in that** the predeterminable number of ventricular events (13, 16) in the reference signal (15) lies in a range from 2 to 100.

3. Implantable medical device according to claim 1 or 2, **characterized in that** a pacing event (19) being visible in the supervised signal is not counted as ventricular event (13, 16).

4. Implantable medical device according to any of the preceding claims, **characterized in that** the computer-readable program causes the processor (32) to generate, in case that no ventricular event (13, 16) has been detected in the reference signal (15) during a predeterminable first time period, a timeout event that is deemed to be a ventricular event (13, 16) in the reference signal (15).

5. Implantable medical device according to claim 4, **characterized in that** the computer-readable program causes the processor (32) to repeatedly generate the timeout event with a predeterminable repetition rate in case that no ventricular event (13, 16) has been detected in the reference signal (15) during the predeterminable first time period.

6. Implantable medical device according to any of the preceding claims, **characterized in that** the computer-readable program causes the processor (32) to store the number of ventricular events (13, 16) detected in the supervised signal (18) during a single interval of the reference signal (15) as value in a ring buffer (305).

7. Implantable medical device according to claim 6, **characterized in that** the ring buffer (305) is configured to store a number of values that corresponds to the predeterminable number of ventricular events (16, 13) in the refence signal (15).

8. Implantable medical device according to any of the preceding claims, **characterized in that** the computer-readable program causes the processor (32) to store the latest 5 to 180 seconds of the reference signal (15) and/or of the supervised signal (18) prior to determining whether the deviation exceeds the threshold.

9. Implantable medical device according to any of the preceding claims, **characterized in that** the computer-readable program causes the processor (32) to store the next 1 to 60 seconds of the reference signal (15) and/or of the supervised signal (18) upon determining whether the deviation exceeds the threshold.

10. Implantable medical device according to any of the preceding claims, **characterized in that** the memory unit (33) comprises i) a ring buffer (305) for short-term caching the reference signal (15) and/or the supervised signal (18) and ii) a long-term memory, wherein storing of at least a part (21, 22) of the reference signal (15) and/or of the supervised signal (18) comprises writing the part (21, 22) of the reference signal (15) and/or of the supervised signal (18) to be stored into the long-term memory.

11. Implantable medical device according to any of the preceding claims, **characterized in that** the counter is a day counter that serves for storing a number of deviations per day that is bigger than the threshold.

12. Implantable medical device according to any of the preceding claims, **characterized in that** the computer-readable program causes the processor (32) to use i) the right ventricular electric signal as reference signal (15) and the left ventricular electric signal as supervised signal (18) as well as ii) the left ventricular electric signal as reference signal (15) and the right ventricular electric signal as supervised signal (18) in a parallel evaluation of an identical temporal section of the right ventricular electric signal and the left ventricular electric signal.

13. Method for operating an implantable medical device (1) for stimulating a human or animal heart, in particular an implantable medical device (1) according to any of the preceding claims, **characterized by** the following steps:
a) detecting a right ventricular electric signal with a first electrode (5), the detected right ventricular electric signal comprising a plurality of right ventricular events (13);
b) detecting a left ventricular electric signal with a second electrode (9), the detected left ventricular electric signal comprising a plurality of left ventricular events (16);
c) defining one of the right ventricular electric signal and the left ventricular electric signal as reference signal (15) and the other as supervised signal (18);
d) counting a number of ventricular events (13, 16) in the supervised signal (18) occurring during a predeterminable number of ventricular events (16, 13) in the reference signal (15);
e) determining a deviation between the number of ventricular events (13, 16) in the supervised signal (18) and the predeterminable number of ventricular events (16, 13) in the reference signal (15);
f) if the deviation between the number of ventricular events (13, 16) in the supervised signal (18) and the predeterminable number of ventricular events (16, 13) in the reference signal (15) is bigger than a threshold, i) perform a storing of at least a part (21, 22) of the reference signal (15) and/or of at least a part of the supervised signal (18) in the memory unit (33) to allow a later evaluation of the stored part (21, 22) of the reference signal (15) and/or of the supervised signal (18) and/or ii) increment a counter for each deviation that is bigger than the threshold and to store the incremented counter and/or iii) sending a notification via the data communication unit (36) to an external device or remote system.

14. Method according to claim 13, **characterized in that** the counter is a day counter that serves for storing a number of deviations per day that is bigger than the threshold, and **in that** a progression of the day counter over a course of days is monitored and/or analyzed.
